# EUROPEAN PATENT SPECIFICATION

(11) **EP 0 966 535 B1**
(45) Date of publication and mention of the grant of the patent: **08.12.2004**
(21) Application number: 98900843.8
(22) Date of filing: 13.01.1998
(51) Int. Cl.: C12N 15/40, C07K 14/18, A61K 39/12, C07K 16/10

(54) **EPITOPES OF THE PROTEIN PRE-M/M OF THE DENGUE VIRUS, SYNTHETIC PEPTIDES**
EPITOPE VOM PRE-M/M PROTEIN AUS DEM DENGUE-VIRUS, SYNTHETISCHE PEPTIDE
EPITOPES DE LA PROTEINE PRE-M/M DU VIRUS DE LA DENGUE, PEPTIDES SYNTHETIQUES

(30) Priority: 15.01.1997 CU 1397
(43) Date of publication of application: 29.12.1999
(73) Proprietor: CENTRO DE INGENIERIA GENETICA Y BIOTECNOLOGIA (CIGB), Ciudad de la Habana 10600 (CU); Instituto de Medicina Tropical "Pedro Kouri" IPK, Ciudad de la Habana 11100 (CU)
(72) Inventor: VAZQUEZ RAMUDO, Susana, La Lisa, Ciudad de la Habana 11500 (CU); GUZMAN TIRADO, Guadalupe, Ciudad de la Habana 11300 (CU); GUILLEN NIETO, Gerardo Enrique, Plaza, Ciudad de la Habana 10400 (CU); PARDO LAZO, Orlando Luis, Ciudad de la Habana 10700 (CU); CHINEA SANTIAGO, Glay, Ciudad de la Habana 10600 (CU); PEREZ DIAZ, Ana Beatriz, Plaza, Ciudad de la Habana 10400 (CU); PUPO ANTUNEZ, Maritza, Ciudadde la Habana 11000 (CU); RODRIGUEZ ROCHE, Rosmari, Bauta, La Habana 32400 (CU); REYES ACOSTA, Osvaldo, 10 de Octubre, Ciudad de la Habana 10700 (CU); GARAY PEREZ, Hilda Elisa, Ciudad de la Habana (CU); PADRON PALOMARES, Gabriel, Ciudad de la Habana 10600 (CU); ALVAREZ VERA, Maylin, 10 de Octubre, Ciudad de la Habana 10700 (CU); MORIER DIAZ, Luis, Arroyo Naranjo,Ciudad de la Habana 11900 (CU); PEREZ INSUITA, Omaida, Ciudad de la Habana 11500 (CU); PELEGRINO MARTINEZ DE LA COTERA, José Luis, La Lisa, Ciudad de la Habana 11500 (CU)
(74) Representative: Prins, Adrianus Willem, Mr. Ir.
(86) International application number: PCT/CU1998/000001
(87) International publication number: WO 1998/031814

(56) References cited:
- IRIE K ET AL: "SEQUENCE ANALYSIS OF CLONED DENGUE VIRUS TYPE 2 GENOME (NEW GUINEA -C STRAIN)" GENE, vol. 75, 1989, pages 197-211, XP000601610
- ZHAO, B. ET AL.: "Cloning full-length Dengue type 4 viral DNA sequences: analysis of genes coding for structural proteins" VIROLOGY, vol. 155, 1986, ORLANDO US, pages 77-88, XP002062826
- GRUENBERG A ET AL: "PARTIAL NUCLEOTIDE SEQUENCE AND DECUDED AMINO ACID SEQUENCE OF THE STRUCTURAL PROTEINS OF DENGUE VIRUS TYPE 2, NEW GUINEA C AND PUO- 218 STRAINS" JOURNAL OF GENERAL VIROLOGY, vol. 69, no. PART 06, June 1988, pages 1391-1398, XP000600928
- BRAY M.; LAI C.: 'Dengue virus premembrane and membrane proteins elicit a protective immune response' VIROLOGY vol. 185, 1991, pages 505 - 508

## Description

### Technical branch

The present invention is in the field of biotechnology and relates to recombinant DNA techniques, in particular to the production of synthetic peptides coding for pre-M/M protein of Dengue virus serotype 2 and chimeric proteins which contain epitopes of pre-M/M protein of Dengue virus serotype 2 and 4.

The technical objective is to identify Pre-M/M neutralizing and protective epitopes, cross reactive for all dengue virus serotypes to obtain an immunogen for human vaccination.

### Previous technique

Dengue virus belongs to the Flavivirus genus, family Flaviviridae (Westaway, E.G. *et al*. 1985. Flaviviridae. **Intervirol.** 24 p.183). It is an enveloped virus with a single RNA chain of positive polarity as genetic material, which codes for a polyprotein processed co- and post- transductionally by cellular and viral proteases.

There are two structural proteins in the viral membrane: E (envelope) and M (membrane), while there are several copies of the other structural protein, C (capside) forming the isometric nucleocapside. Besides, at least seven non-structural proteins have been identified (NS1, NS2a, NS2b, NS3, NS4a, NS4b, NS5).

Glycoproteins E and NS1 are individually able to offer active and passive protection against the homologous serotype of Dengue virus, while the highly conformational complexity of the relevant epitopes is preserved. For this reason, recombinant eukaryotic cellular systems have been mainly selected for the immunological evaluation of these proteins, for example vaccinia virus (Bray, M. *et al*. 1989. Mice immunized with recombinant Vaccinia virus expressing dengue-4 structural proteins with or without nonstructural protein NS1 are protected against fatal dengue virus encephalitis. **J. Virol.** 63 p.2853) and baculovirus (Zhang, Y. M. *et al*. 1988. Immunization of mice with dengue structural proteins and nonstructural protein NS1 expressed by baculovirus recombinant induces resistance to dengue virus encephalitis. **J. Virol.** 62 p.3027).

The small protein M (8 kDa) is synthesized as a glycosylated precursor named pre-M (22 kDa approximately), which suffers a late endoproteolytic cleavage just before or after the liberation of the virus from the infected cell (Murray, J. M. *et al*. 1993. Processing of the dengue virus type 2 proteins prM and C-prM. **J. Gen. Virol.** 74 p.175). The cleavage, which is probably done by a cellular protease, seems to happen in the post-Golgi acidic vesicles, being inhibited by agents that destabilize the low pH of these vesicles (Randolph, V. B. *et al*. 1990. Acidotropic amines inhibit proteolytic processing of Flavivirus prM protein. **Virol**. 174 p.450). The pre-fragment has been identified in vitro only in the extracellular medium, its destiny in vivo remains unknown (Murray, J. M. *et al*. 1993. Processing of the dengue virus type 2 proteins prM and C-prM. **J. Gen. Virol.** 74 p.175).

It is thought that the function of pre-M during the Flavivirus exocytic passage is to avoid the activation of the fusogenic membrane domain of E with the acidic pH of the environment (Randolph, V. B. *et al*. 1990. Acidotropic amines inhibit proteolytic processing of Flavivirus prM protein. **Virol.** 174 p.450); if this event happens, then the viral liberation will be prevented. In fact, it has been determined that pre-M and E interact in the immature intracellular virions (Wengler, G. y Wengler, G. 1989. Cell-associated West Nile flavivirus is covered with E+pre-M protein heterodimers which are destroyed and reorganized by proteolytic cleavage during virus release. **J. Virol.** 63 p.2521), and that the native conformation of E is only acquired in the presence of pre-M (Konishi, E. y Mason, P.W. 1993. Proper maturation of the Japanese encephalitis virus envelope glycoprotein requires cosynthesis with the premembrane protein. **J. Virol.** 67 p.1672). In addition, already liberated virions that only have pre-M in their membranes show, in general, a lower infectivity than completely mature virions (Wengler, G. y Wengler, G. 1989. Cell-associated West Nile flavivirus is covered with E+pre-M protein heterodimers which are destroyed and reorganized by proteolytic cleavage during virus release. **J. Virol.** 63 p.2521), in which although M and pre-M are present, the former is predominant.

Pre-M and M offer an active protection when they have been expressed in recombinant vaccinia virus, but this does not happen with the pre-fragment (Bray, M. y Lai, C.-J. 1991. Dengue virus premembrane and membrane proteins elicit a protective immune response. **Virol.** 185 p.505), besides the combination of pre-M or M with glycoprotein E in the same recombinant Vaccinia virus gives in general levels of protection higher than those reached by each protein individually. Similarly, certain antibodies against pre-M/M are able to protect passively in mice (Kaufman, B. M. *et al*. 1989. Monoclonal antibodies for dengue virus prM glycoprotein protect mice against lethal dengue infection. **Am J. Trop. Med. & Hyg.** 41 p.576).

The use of synthetic peptides has allowed to establish the molecular basis of antigenicity according to spatial conformation and the immunological properties of the antigen involved [Arnon, R. y Sela, M. 1985. Synthetic Vaccines : present and future. **Ann. Inst. Pasteur/Immunol** 136 D, 271-282]. The synthetic peptides as anti-dengue vaccine subunits will allow to include in the final formulation only the protective epitopes that do not cause immune-amplification (Halstead, S.B., y O'Ruourke, E.J. 1977. Dengue viruses and mononuclear phagocytes. I. Infection enhancement by non-neutralizing antibody. **J. Exp. Med.** 146 p.201; Halstead, S.B. 1979. In vivo enhancement of dengue virus infection in rhesus monkeys by passively transferred antibody. **J. Infect. Dis.** 140 p.527), or alternatively, to include protective peptides of each of the four serotypes. The characterization of the antigenic determinants of E and NS1 has been carried out successfully. However, there are no similar studies on the also important protein pre-M/M, that is why the results of this paper are a first step in that direction.

The efforts to express the flaviviral proteins pre-M, M and E in E. coli have not always been successful (Chambers, T. J. *et al*. 1990. Production of yellow fever virus proteins in infected cells: identification of discrete polyprotein species and analysis of cleavage kinetics using region-specific polyclonal antiserum. **Virol.** 177 p.159; Yan, B.-S. *et al*. 1994. Truncating the putative membrane association region circumvents the difficulty of expressing hepatitis C virus protein E1 in *Escherichia coli*. **J. Virol. Meths.** 49 p.343). Apparently, the hydrophobic regions these proteins have in the C-terminal part are the cause of the low or undetectable heterologous expression levels (Yan, B.-S. *et al*. 1994. Truncating the putative membrane association region circumvents the difficulty of expressing hepatitis C virus protein E1 in *Escherichia coli.* **J. Virol. Meths.** 49 p.343).

The expression of these proteins (as well as NS1) in E.coli, in general has been obtained by fusion (fragmented or not) to other bacterial proteins (e.g. β-galactosidase (Cane, P.A. y Gould, E.A. 1988. Reduction of yellow fever mouse neurovirulence by immunization with a bacterially synthesized non-structural protein (NS1) fragment. **J. Gen. Virol.** 69 p.1241), TRPE (Megret, F. *et al*. 1992. Use of recombinant fusion proteins and monoclonal antibodies to define linear and discontinuous antigenic sites on the Dengue envelope glycoprotein. **Virol.** 187 p.480) and the protein A of *Staphylococcus aureus* (Murray, J. M. et *al*. 1993. Processing of the dengue virus type 2 proteins prM and C-prM. **J. Gen. Virol.** 74 p.175). In these fusion proteins most of the relevant conformational epitopes are absent, because though the antisera generated against them can recognize the whole virus, they are not able to neutralize it or to inhibit its hemagglutinating properties (Megret, F. *et al*. 1992. Use of recombinant fusion proteins and monoclonal antibodies to define linear and discontinuous antigenic sites on the Dengue envelope glycoprotein. **Virol.** 187 p.480). However, recent reports show that the solubility of the fusion proteins, and as a consequence, the use of non-denaturalizing methods for its purification, may preserve most of the neutralizing (Seif, S.A. *et al*. 1995. Finer mapping of neutralizing epitope(s) on the C-terminal of Japanese encephalitis virus E-protein expressed in recombinant *Escherichia coli* system. **Vaccine** 13 p.1515) and protective (Srivastava, A.K. *et al*. 1995. Mice immunized with a dengue type 2 virus E and NS1 fusion protein made in *Escherichia coli* are protected against lethal dengue virus infection. **Vaccine** 13 p.1251) epitopes they possess.

In the case of pre-M, its pre-domain has 6 cysteins involved in 3 disulfide bridges, as well as an N-glycosylation site at the asparagine 69. The structure of E and NS1 is even more complicated; it involves 6 disulfide bridges and several N-glycosylation sites. However, the small ectodomain of M is apparently free of those conformational complexities because it does not have cysteins, and it is not glycosylated in its natural form.

The insertion of heterologous fragments in permissive areas of immunogenic proteins whose topology is more or less known and immunization with these fusion products is a complementary alternative to the use of synthetic peptides. Both strategies allow defining the presence of sequential B cell, as well as T cell epitopes. The biological importance of these epitopes can be experimentally evaluated to decide whether to include them or not in certain vaccine preparation.

### Detailed description of the invention.

A number of five peptides from Pre-M/M protein of Dengue 2 virus which cover 58 % of the aminoacid sequence (97/166 AA) were chemically synthesized. They corresponded to the amino acid sequences 3-31; 45-67; 57-92; 69-93; and 103-124, which were subsequently named B 19-6; B 20-2; B 19-5; B 20-1; B 20-3 respectively.

Peptides conjugated or not conjugated to a carrier protein, were inoculated in Balb/c mice. The sera obtained after immunization with the conjugated peptides were tested by in vitro neutralization by reduction of the number of plaques and by ELISA. We also studied the active protection against a Dengue 2 viral challenge in the immunized mice.

In the case of mice immunized with the non-conjugated peptides, the antibody response was evaluated by ELISA and the proliferative response of spleen T lymphocytes against Dengue 2 virus was evaluated too.

Fusion proteins were also obtained, and two of the four regions covered by peptides (1-42 and 92-133) were inserted to them and were expressed in E.coli bacteria. Immunization with these fusions will complement the results obtained with the synthetic peptides.

The presence of B cell epitopes in both mice and humans was demonstrated as the peptides were recognized by antibodies from the immunized mice and by sera from patients who had the clinical and serological diagnosis of Dengue virus, using ELISA in both cases. Peptides 19-6 and 20-3 were able to induce neutralizing antibody production against the four Dengue virus serotypes.

Virus-specific proliferative responses were demonstrated in mice immunized with non-conjugated peptides 19-6 and 19-5.

Mice immunized with conjugated peptides 19-6, 20-1, and 19-5 showed a statistically significant level of protection when they were challenged with Dengue 2 virus.

Thus, the presence of sequential epitopes in Pre-M/M protein of Dengue virus 2 was demonstrated, as well as their relevance in the immune response against these flaviviruses.

### EXAMPLES OF REALIZATION

### EXAMPLE 1. Prediction of antigenic regions and of T-cell epitopes of pre-M/M protein of Dengue virus.

Different theoretical methods were applied to predict the antigenic regions in the pre-M/M protein of Dengue-2 virus. These regions are those more likely to be recognized by antibodies obtained against the viral proteins, as well as to generate antibodies that recognized the original proteins. Some methods to predict T-cell epitopes were applied.

Five initial peptides that have possible B- and T- cell epitopes were found (4 in pre- and 1 in M). The study of the antigenic structure of these proteins and the experimental determination of possible immunologically important peptides was based on this finding.

### 1.1 Predictions of humoral antigenicity.

Methods used to predict the antigenicity were based on the aminoacidic sequence, since neither the tridimensional structure of the pre-M/M protein of Dengue virus has been determined experimentally nor is there a significant similarity at sequence level with any protein of known tridimensional structure.

The A 15 strain of Dengue 2 isolated in Cuba in 1981 (Kouri, G. *et al*. 1986. Hemorrhagic dengue in Cuba: history of an epidemic. **Bull. P.A.H.O** 20 p.24) was used to accomplish this example. The potentially antigenic regions were selected according to the following criteria:
a) regions of high antigenic propension according to different prediction methods based on hydrophilicity (Hoop, T.P. y Woods, K.R. 1981. Prediction of protein antigenic determinants from amino acid sequences. **Proc. Natl. Acad. Sci. USA** 78 p.3824; Parker, J.M.R. *et al*. 1986. New hydrophility sale derived from HPLC peptide retention data: correlation of predicted surface residues with antigenicity and X-ray derived accessible sites. **Biochemistry** 25 p.5425), flexibility (Karplus, P.A. y Schultz, G.E. 1985. Prediction of chain flexibility in proteins. A tool for the selection of peptide antigens. **Naturwissenschaften** 72 p.212) and accessibility (Emini, E.A. *et al*. 1985. Induction of hepatitis A virus-neutralizing antibody by a virus specific synthetic peptide. **J. Virol.** 55 p.836).
b) regions with high possibility of forming loops and turns according to predictions of secondary structure that use PHD (Rost, B. y Sander, C. 1993. Prediction of protein secondary structure at better than 70% accuracy. **J. Mol. Biol.** 232 p.584; Rost, B. y Sander, C. 1994. Combining evolutionary information and neural networks to predict protein secondary structure. **Proteins** 19 p.55; Rost, B. y Sander, C. 1994. Conservation and prediction of solvent accessibility in protein families. **Proteins** 20 p.216).
c) regions of high variability that include or not insertions/suppressions in respect to other flaviviruses, as well as potential regions of glycosylation in other flaviviruses that are used or not in Dengue virus.

### a) Antigenicity profiles.

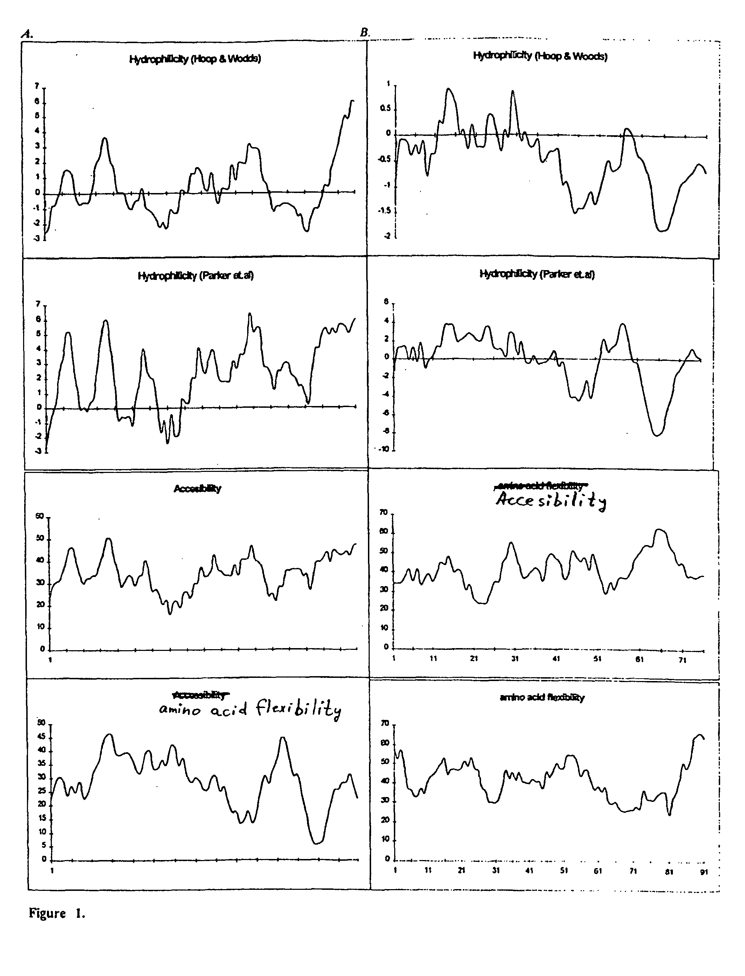

Figure 1 shows the profiles that are obtained when applying to the pre- and M segments 4 properties of the amino acids related to the antigenicity (Pre-M at the left, M at the right.)

In the pre- region there are high hydrophilicity and accessibility values in the regions that have the residues 6-9, 16-21, 28-31, 42-47, 58-65 and 82-91. In the M protein, a vast hydrophobic region exists between the residues 41-76, which corresponds to the transmembrane helices that are thought not to be exposed to the immune system. In the small ectodomain of M (residues 1-40) the region of major hydrophilicity/accessibility extends between the amino acids 13-31, especially at its beginning (AA 13-16).

### b) Predictions of secondary structure.

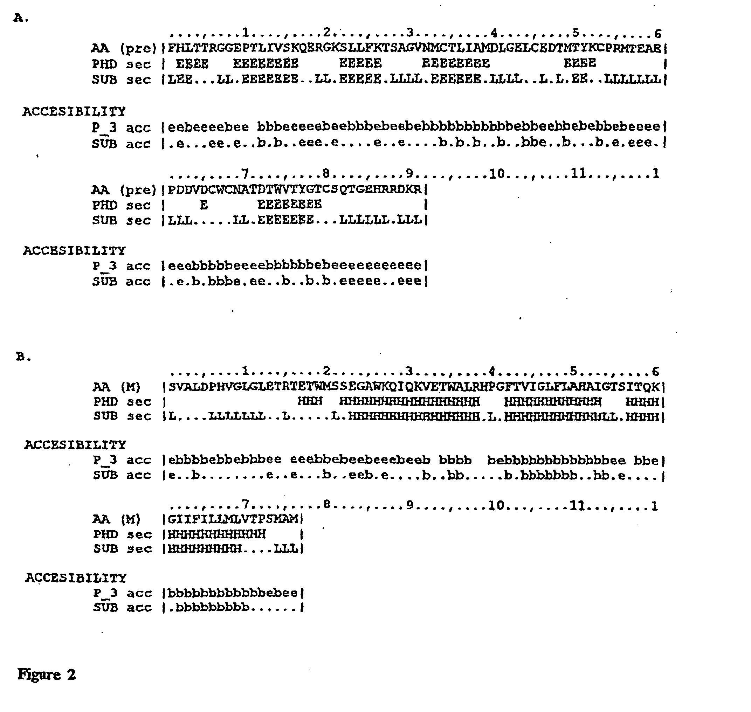

Figure 2 shows the predictions of secondary structure and accessibility of the pre- and M segments according to the PHD program. The results of the predictions show that many potentially antigenic regions (according to the profiles of Figure 1) are predisposed to form loops/β-turns with exposed residues at the surface of the protein. The formation of transmembrane helices is predicted for the region between aminoacids 41-76 of protein M, and this is in accordance with the hydrophobic character of this region and suggests that the antigenic peptides of M are mainly in the ectodomain (1-40).

### c) Alignments of sequences of pre- and M protein of Dengue and other Flavivirus. Variability and glycosylation.

In general, regions that are not exposed to the solvent are more conserved in families of homologous proteins. Therefore, regions of higher variability have a higher probability to be exposed.

In the case of viruses, variability is also an escape mechanism for the immunological pressure; of course this does not exclude that some conserved regions might be antigenic or that there could be conserved regions at the surface. The analysis of sequences of pre- and M regions of 15 isolates of the 4 serotypes of Dengue virus shows that at least 69% of the residues is strictly conserved. The more important variable residues are in the positions 28-30, 55-59, 69-72 and 80-83 of pre-, as well as in positions 27-30 of M. In general, these zones correspond to the maxima of the antigenic profiles of Figure 1.

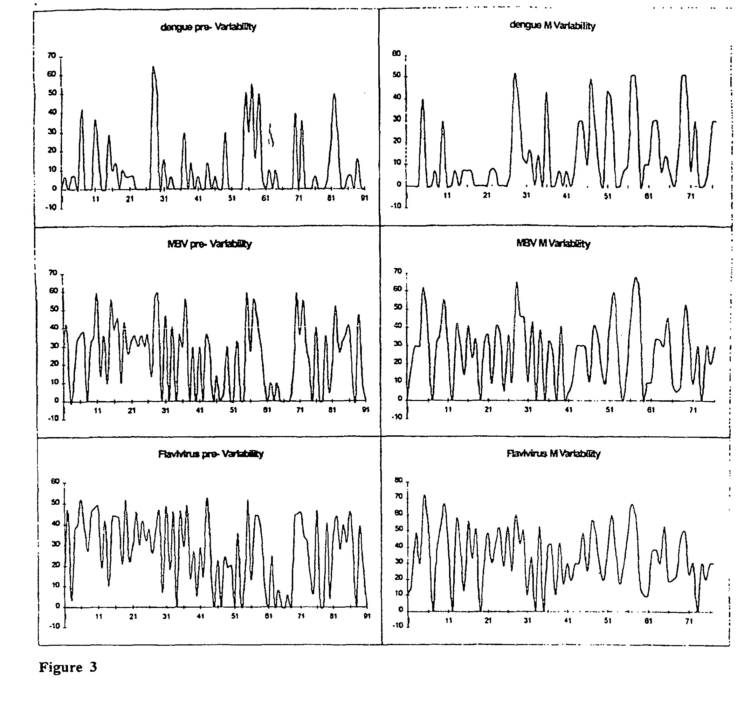

The comparison of the sequences of these regions in more than 30 flaviviral isolates shows that the 1-33 region of pre- is highly variable, with possible loops predisposed to insertions/suppressions (in the positions 8 and 30) and several potential sites of N-glycosylation. On the contrary, the variability is lower in the 33-91 domain of pre-; there are several positions strictly conserved in all flaviviruses, for example: 6 cysteins forming 3 disulfide bridges, at least 5 acidic residues in the region 40-65, as well as the basic sequence 87-91, behind which the endoproteolytic cleavage occurs just before or during the liberation of the mature virus. (Figure 3)

Asn-69, conserved residue in the antigenic Dengue complex is the only N-glycosylation site of pre-M/M protein of the complex. However, in the Flaviviridae family this region is in a possible exposed loop of high variability. At the same time the pre-M/M residues of Dengue virus corresponding to the potential N-glycosylation sites in other Flaviviruses (for example, AA 14 in JE, SLE, MVE YF and AA 32 in LI, LAN, YF TBE) are β-turns close to zones considered as antigenic.

### 1.2 Prediction of T cell epitopes.

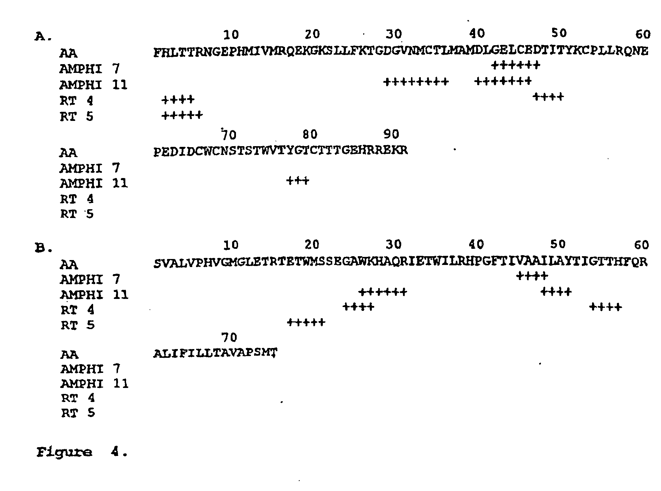

The prediction was done by two independent methods: the Rothbard and Taylor pattern method (Rothbard, J.B. y Taylor, W.R. 1988. A sequence pattern common to T-cell epitopes. **EMBO J.** 7 p.93.) and the determination of fragments with propensity to form alpha-helix structures (AMPHI 7 and 11) (Margalit, H. *et al*. 1987. Prediction of immunodominant helper T cell antigenic sites from the primary sequence. **J. Immunol.** 138 p.2213.). Results are shown in Figure 4.

### 1.3 Peptides proposed for the identification of relevant epitopes.

The determination of neutralizing and protective peptides in general is very important for the development of more efficient vaccines, and peptides from regions of high antigenic propension are very useful for their identification; especially of those of linear nature.

Table 1 shows a set of peptides that include regions predisposed to have B and T cell epitopes (according to the several predictive methods used in this example) of pre-M/M protein of D2 virus. If the validity of that prediction is demonstrated experimentally, the immunological important epitopes of each region will be localized accurately by the design of smaller peptides in each of them.

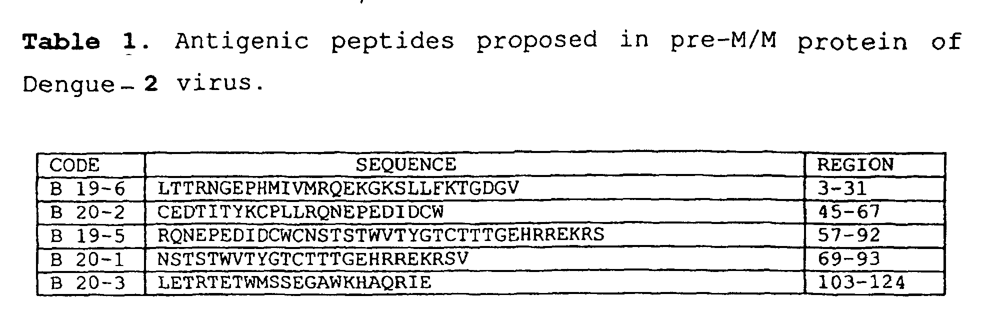

### EXAMPLE 2. Chemical synthesis of oligopeptides and oligonucleotides.

### 2.1. Synthesis of oligopeptides.

All peptides were synthesized using a Boc- strategy in solid phase on the p-methyl-benzhydrylamine resin (resin MBHA, BACHEM, Switzerland).

The protected amino acids were obtained from BACHEM. The protection of reactive groups of the amino acid chain was: Arg (Tos), Asp (OBzl), Cys (4-Me-Bzl), Glu (OBzl), Lys (2-Cl-Z), Trp (CHO), Tyr (Cl₂-Bzl), Thr (Bzl). Asn, Gln and Pro were used without protection in the lateral chains.

Removal of the Boc-amino protective group was carried out using 37.5% trifluoracetic acid in dichloromethane. Activation with diisopropyl-carbodiimide (DIC) in situ was used for the coupling reaction of each residue, except for amino acids Asn and Gln, which were activated using DIC and 1- hydroxybenzotriazole in N,N-dimethylformamide.

Final deprotection and peptide liberation from the resin was accomplished in special equipment. The procedure used is known as Low-High HF.

During the first part of the procedure (Low HF), the protected-resin system was treated with HF (25%): DMS (65%): p-cresol (10%) during 120 minutes at 0°. The mixture was replaced by HF (25%): DMS (60%): EDT (10%): p-cresol (5%) in the case of Trp-containing peptides. Subsequently, the resin-peptide was washed several times with diethyl ether, dichloromethane and 2-propanol, and was vacuum-dried.

During the second part of the procedure (High HF), the resin-peptide was treated with HF (90%): anizole (10%) during 60 minutes at 0°.

The raw product was washed with ether, then was extracted with 30% acetic acid in water and finally was lyophilized.

Peptides were characterized by RP-HPLC in a BAKER C-18 (4.6 x 100 mm) column and by mass spectrometry using FAB as ionization method in a JEOL HX- 110 HF equipment.

The aminoacid sequence as well as its location in the pre-M/M protein of Dengue virus is shown in Table 1.

### 2.2. Synthesis of oligonucleotides.

Oligonucleotides were automatically synthesized in the equipment Gene Assembler Plus, according to the phosphoramidite method.

The sequence of the six oligonucleotides is shown in Table 2.

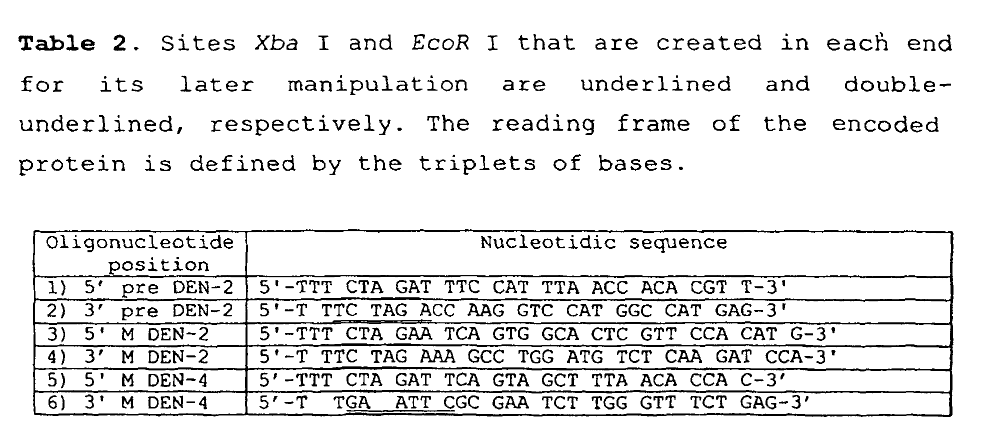

### EXAMPLE 3. Coupling of peptides to a carrier protein and immunization scheme.

### 3.1 Coupling of peptides to BSA.

The coupling of peptides was performed as follows:
1. Activation of BSA: Drop by drop and with shaking, 80 µl of a solution of the bifunctional reagent m-maleimidobenzoyl-N-hydroxysuccinimide ester (MBS) [5 µg/µl] in dimethylformamide, were added to a solution of 2.8 mg of bovine albumin fraction V (BSA) in 250 µl of PBS. Then, it was kept in agitation at room temperature during 30 minutes, and the mixture was passed through a PD10 column.
2. Coupling of the peptide to the activated BSA: a solution of 1 mg of peptide dissolved in 300 µl of PBS was added to the activated BSA solution drop by drop and with shaking. It was kept at room temperature during 3 hours, and concentration was determined by the Lowry method.

### 3.2 Immunization scheme

The immunization scheme of peptides linked to BSA is the following:
Male Balb/c mice of 4-6 weeks old were immunized with 50 µg of the peptide-BSA conjugate intraperitoneally. In addition, two immunization schemes were performed, one with BSA and the other with PBS. A total of 4 inoculations were carried out, each of them 15 days apart. The Freund's Complete Adjuvant was used in the first doses, and the Freund's Incomplete Adjuvant in the others. A blood sample was extracted from the retro-orbital vein seven days after the last inoculation.
The obtained sera of each scheme were placed at -20°C to be used later.

### EXAMPLE 4. In vitro plaque-reduction neutralization test.

Neutralization technique was carried out according to Morens (Morens, D.M. *et al*. 1985. Simplified plaque reduction neutralization assay for dengue viruses by semimicro methods in BHK-21 cells: Comparison of the BHK suspension test with standard plaque reduction neutralization. **J. Clin. Microbiol.** 22 p.250).

Dilutions of anti-peptide sera.and of anti-BSA controls and negative sera from 1/10 to 1/640 were prepared. Each dilution of sera was put into contact with a dilution of the virus (strain A 15 of Dengue 2) having 15-20 PFU/50 µl.

The mixture was incubated at 37°C during 1 hour. A total of 50 µl of each mixture were added in triplicate to BHK-21 cells in plates of 24 wells, and they were incubated in a CO₂ incubator at 37°C during 4 hours. Then, 0.5 ml of carboxymethylcellulose-containing medium were added, and it was incubated again for several days considering the viral serotype used. After these days tinction and the count of lysis plaques produced by the virus were carried out.
Titer was expressed in each case as the dilution at which 50 % of plaque number reduction was obtained.
The results are shown in Table 3.

**Table 3.**

| PRNT of the anti-peptide sera against 19-6 and 20-3. | | | | |
|---|---|---|---|---|
| Anti-peptide | Neutralizing titer for each serotype | | | |
| | D₁ | D₂ | D₃ | D₄ |
| B 19-6 | 1/100 | 1/180 | 1/60 | 1/160 |
| B 20-3 | 1/110 | 1/80 | 1/80 | 1/80 |

### EXAMPLE 5. Identification of T cell epitopes.

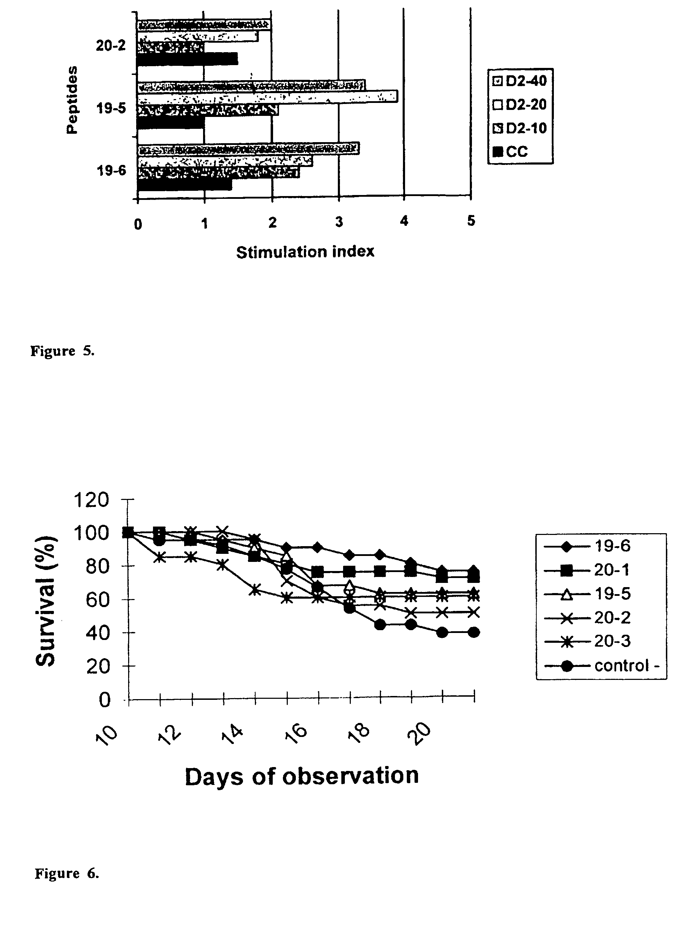

The presence of T cell epitopes in the peptides of pre-M was evaluated throughout the study of anti-peptide antibody response elicited in free peptides (non-conjugated) immunized mice. Primed animals demonstrated higher serum antibody production in response to a booster dose of antigen when compared to the response in naive animals. These results confirm the existence of B cell epitopes in these peptides and show that these sequences contains T cell epitopes, which are able to stimulate Th activity *in vivo* to improve the titers of antibody response.
Virus-specific proliferative responses of spleen T lymphocytes were demonstrated in peptide immunized BALB/c mice. T cells from 19-6 and 19-5 immunized mice proliferated in an *in vitro* blastogenesis assay when they were cultured with the dengue 2 virus. However, the 20-2 peptide did not elicit a significant proliferative response against the virus. It could contain a T cell cryptic epitope, being recognized in the free form of peptide but not like a result of the immunodominant epitopes presentation and processing of the virus in a natural infection (Figure 5).

### EXAMPLE 6. Protection assay.

Mice were challenged 7 days after the last immunization by intracraneal injection with a dilution of 1/2500 (corresponding to 100 LD50 lethal doses) of live, mouse-adapted dengue-2 virus (strain A15). Mice were observed for up to 21 days for morbidity and mortality. Data were tested for statistical significance using Fisher's test. The percent survival in peptide immunized and control animals are shown in Figure 6. The level of protection induced for the peptides 19-5, 19-6 and 20-1 was statistically significant (p< 0,05).

### EXAMPLE 7. Indirect ELISA to detect anti-peptide antibodies.

### Human sera:

Peptides 19-6, 20-1, 20-2, 20-3 were fixed to the plates in a 10 µg/ml concentration in coating buffer, then they were incubated at 4°C overnight. Sera were added diluted 1/200 in PBS-Tween 20. Finally, a conjugate of anti-total human immunoglobuline and peroxidase was added, and subsequently, the substrate (orthophenylendiamine, H₂O₂, 0.05 M phosphate citrate buffer, pH 5) was added. Reading was carried out in an ELISA reader at 492 nm and the cut-off value for each peptide was determined.

The sera used were from subjects having viral clinical infection that was serologically diagnosed as Dengue by the techniques of hemagglutination inhibition (Clarke, D.H. y Casals, J. 1958. Techniques for hemagglutination and hemagglutination - inhibition with Arthropod Borne Virus. **Am.** **J. Trop. Med. Hyg.** 7 p.561) and ELISA of inhibition (Vázquez, S. , Fernández, R. 1989. Utilización de un método de ELISA de Inhibición en el diagnóstico serológico de dengue. **Rev. Cub. Med. Trop.** 41(1) p18-26) for total anti-dengue antibodies. The study included 118 sera from patients of the epidemics which occurred in Cuba 1981, Panama 1994 and Costa Rica 1994. Dengue virus 2 was isolated in these epidemics, besides serotype 1 and 4 in Costa Rica; they were classified according to the titers of hemagglutination inhibiting antibodies in cases of primary and secondary infections.

46.6% of the sera were positive to the 4 peptides used. Percentages of positivity of 56.8%, 79.6%, 77.1% and 83.1% to peptides B 19-6, 20-1, 20-2, and 20-3 were obtained respectively.

The average of the reactivity index, calculated by the quotient optical density of the sample/cut-off value, for each peptide was 1.07, 1.52, 1.57 and 1.49, respectively.

### Mice sera:

The indirect ELISA used was as described above but using an anti-mouse Ig conjugated to peroxidase. Antibody titers obtained in the anti-peptide sera were generally above 1/10,000.

### EXAMPLE 8. Insertion of pre-M/M fragments in the P64k protein of Neisseria meningitidis.

In this example we expressed fragments of the pre-M/M protein of Dengue 2 (A 15 strain) and of Dengue 4 (814669 strain) (Zhao, B. *et al*. 1986. Cloning full-length dengue type 4 viral DNA sequences: analysis of genes coding for structural proteins. **Virol.** 155 p.77) inserted in a *N*. *meningitidis* protein previously characterized in our group (Silva, R. *et al*. 1992. Nucleotide sequence coding for an outer membrane protein from *Neisseria meningitidis* and use of said protein in vaccine preparations. **European Patent 0 474 313,** 1997): P64k, which has proven to be highly immunogenic in several animal models. Besides, the level of expression of P64k in *E*. *coli* reaches more than 30% of the total protein of the bacteria.

P64k protein (64 kDa) of dimeric nature, has two functional domains in each subunit: one with lipoic acid-binding activity (1-100) and the other with lipoamide-dehydrogenase activity (117-594). Both have been identified as relatively independent conformational domains by X-ray crystallography (Li de la Sierra, I. *et al*. 1994. Crystallization and preliminary X-ray investigation of a recombinant outer membrane protein from *Neisseria meningitidis.* **J. Mol. Biol.** 235 p.1154; Li de la Sierra, I. *et al*. 1997. Molecular structure of the lypoamide dehydrogenase domain of a surface antigen from *Neisseria meningitidis.* **J. Mol. Biol.** 269 p. 129).

The former domain was selected (in the aminoacid positions 40-45) to perform the insertions of fragments 1-42 and 92-133 of pre-M/M, because this small domain is more exposed and does not seem to be involved in dimer-formation. This suggested that the global structure of the chimeric protein with respect to the natural P64k would be less altered than if an insertion site were made in the domain 117-594, which, in addition participates directly in the formation of the dimer.

The region coding for amino acids 38-47 (TL**ETDKAT**MD), which include the region of lipoic acid binding of the P64k gene used in the production of fusion proteins, was preliminary changed to TL**DLE**MD. This modification was carried out to avoid the recognition of P64k by the sera of patients having primary biliary cirrhosis, who have self-antibodies against homologous epitopes present in the human mitochondrial dehydrolipoamide acetyltransferase (Tuaillon, N. et *al*. 1992. A lipoyl synthetic octadecapeptide of dihydrolipoamide acetyl transferase specifically recognized by anti-M2 autoantibodies in primary biliary cirrhosis. **J. Immunol.** 148 p.445).

The strategy to produce the two clones is explained below: Fragments Pre-2, M-2 and M-4 were amplified by Polymerase Chain Reaction, using the combination of oligonucleotides 1 and 2, 3 and 4, and 5 and 6, respectively (see Table 2), and using pD-5 plasmid as template. This pD-5 plasmid includes a copy of preM/M gene from Dengue-2 virus (strain A-15) cloned into pBluescript vector (stratagene). DNA bands obtained in each case (120 bp) were digested with Xba I (Pre-2 and M-2) or Xba I/EcoR I (M-4), and they were cloned into the corresponding sites that were artificially created in position 135-145 of P64k gene, cloned into vector pM-92. Besides, a chimeric clone that includes the M-2 and M-4 bands in the already mentioned sites Xba I and EcoR I was generated by triple ligation. Recombinant clones bearing the inserts in the right orientation were identified by restriction analysis and DNA-sequencing.

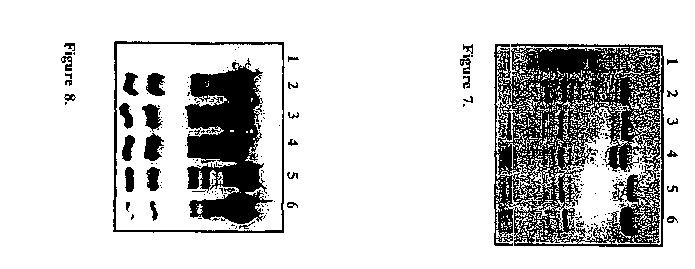

The fusion proteins generated by clones of Pre-2 (pD31), M-2 (pD30), M-2/M-4 (pD33), and M-4 (pD34) were expressed under the promoter of the tryptophan operon (ptrp) in the E. coli strain MM294 (F⁻ end A1 hsdR17 (rₖ⁻ mₖ⁺) sup E44 thi-1 relA1? RfbD1? SpoT1?). All were obtained in the expected sizes and with levels of expression up to 30% of the total proteins of the bacteria, though PD31 protein showed a high instability (Figure 7). All fusion proteins were recognized by some anti-P64k mouse monoclonal antibodies in ELISA (data not shown) and

Western blotting (Figure 8), where a remarkable degradation in the whole-cell extract was detected. The aminoacid sequence of these proteins is shown in the Sequence List.

Mouse immunization with PD33 and PD34 fusion proteins semipurified by a non-denaturalizing protocol, has elicited high titers against them in ELISA (up to 1/100 000), and at the same time antibodies with titers up to 1/4000 in ELISA against the synthetic peptides have been obtained.

### DESCRIPTION OF THE FIGURES.

**Figure 1.** Hydrophilicity, accessibility and flexibility profiles of pre- (A) and M (B) proteins of Dengue virus.
**Figure 2.** Prediction of the secondary structure and of accessibility of pre (A) and M (protein). AA: amino acids. PHD sec: prediction of secondary structure (E= beta, H= helix, L= loop) P- 3 acc. Prediction of accessibility (e=exposed, b= non-exposed). Sub sec (Sub acc) residues in which the prediction of secondary structure (accessibility) is 82.4% (70%) efficacious.
**Figure 3.** Variability profiles of pre- and M proteins. Variability was calculated considering 3 sets of Flavivirus sequences. Dengue: sequences of 15 Dengue isolations. MBV: Flavivirus sequences transmitted by mosquitoes that include Dengue virus, Kunji, West Nile Virus, Murray Valley Encephalitis and Saint Louis Encephalitis. Flavivirus: sequences of more than 30 different flaviviral isolations: (MBV + Yellow fever, Langat, Louping Ill and Tick-Borne Encephalitis).
**Figure 4**. Prediction of T cell epitopes of pre (A) and M (B) proteins: AMPHI 7 (11): prediction of amphipatic segments of 7 (11) residues, positive residues are the central amino acids of an amphipatic block potentially antigenic. RT 4 (5): prediction of antigenic profiles of 4 (5) residues, positive residues are those who fulfill the profiles.
**Figure 5.** Proliferative response to dengue 2 virus antigens (at concentrations of 10, 20 and 40 µg/ml) of spleen T cells from peptides-immunized mice 19-6, 19-5 and 20-2.
**Figure 6.** Percent survival in peptide immunized and control animals. The level of protection induced for the peptides 19-5, 19-6 and 20-1 was statistically significant.
**Figure 7.** 10% SDS-PAGE of MM294 *E. coli* strain transformed with fusion proteins and P64k protein (pM-92 plasmid). Lanes: 1- Untransformed MM294 strain, 2- pM-92/MM294, 3- pD-30/MM294, 4- pD-31/MM294, 5- pD-33/MM294, 6- pD-34/MM294.
**Figure 8**. Western blot using mab 114 against Pbyk expressed by plasmid pM-92 in E. coli strain MM296 Lanes: 1- Untransformed MM294 strain, 2- pM-92/MM294, 3- pD-30/MM294, 4- pD-31/MM294, 5- pD-33/MM294, 6-pD-34/MM294.

## Claims

1. A dengue virus-specific peptide comprising an epitope of Pre-M/M protein of at least one dengue virus serotype, wherein said peptide is amino acids Nos. 3-31, amino acids Nos. 69-93 or amino acids Nos. 103-124, of Pre-M/M.

2. A dengue virus-specific peptide as claimed in claim 1, wherein said peptide comprises an amino acid sequence selected from the group consisting of SEQ ID NO:1, SEQ ID NO:4 and SEQ ID NO:5.

3. A fusion protein comprising a carrier protein and a dengue-specific amino acid sequence comprising an epitope of dengue-2 and/or dengue-4 virus, wherein said dengue-specific amino acid sequence is amino acids Nos. 1-42 or amino acids Nos. 92-134 of the Pre-M/M protein of said virus.

4. A fusion protein as claimed in claim 3, wherein the carrier protein is P64k of *Neisseria meningitidis* and the dengue-specific amino acid sequence is inserted after amino acid No. 42 of P64k.

5. A fusion protein as claimed in claim 4 which comprises a sequence from the group consisting of SEQ ID NO:6, SEQ ID NO:7, SEQ ID NO:8 and SEQ ID NO:9.

6. A dengue-specific antibody which binds to an epitope of Pre-M/M protein of a dengue virus, wherein said epitope is as defined in claim 1 or 2.

7. A pharmaceutical composition against dengue, comprising a dengue virus-specific antibody as claimed in claim 6.

8. A pharmaceutical composition comprising a dengue virus-specific peptide as claimed in claim 1 or claim 2, optionally conjugated to a carrier substance, or comprising a fusion protein as claimed in any one of claims 3-5.

9. A pharmaceutical composition as claimed in claim 8, which contains an adjuvant.

10. A pharmaceutical composition as claimed in claim 8 or 9, which is a vaccine against dengue.

## Patentansprüche

1. Denguevirus-spezifisches Peptid, das ein Epitop des Pre-M/M-Proteins von wenigstens einem Denguevirus-Serotyp umfasst, wobei es sich bei dem Peptid um die Aminosäuren Nr. 3-31, die Aminosäuren Nr. 69-93 oder die Aminosäuren Nr. 103-124 von Pre-M/M handelt.

2. Denguevirus-spezifisches Peptid gemäß Anspruch 1, wobei das Peptid eine Aminosäuresequenz umfasst, die aus der Gruppe ausgewählt ist, die aus SEQ ID Nr. 1, SEQ ID Nr. 4 und SEQ ID Nr. 5 besteht.

3. Fusionsprotein mit einem Trägerprotein und einer Dengue-spezifischen Aminosäuresequenz, die ein Epitop des Dengue-2- und/oder Dengue-4-Virus umfasst, wobei es sich bei der Dengue-spezifischen Aminosäuresequenz um die Aminosäuren Nr. 1-42 oder die Aminosäuren Nr. 92-134 von Pre-M/M-Proteins dieses Virus handelt.

4. Fusionsprotein gemäß Anspruch 3, wobei es sich bei dem Trägerprotein um P64k von *Neisseria meningitidis* handelt und die Dengue-spezifische Aminosäuresequenz nach der Aminosäure Nr. 42 von P64k eingesetzt ist.

5. Fusionsprotein gemäß Anspruch 4, das eine Sequenz aus der Gruppe umfasst, die aus SEQ ID Nr. 6, SEQ ID Nr. 7, SEQ ID Nr. 8 und SEQ ID Nr. 9 besteht.

6. Dengue-spezifischer Antikörper, der an ein Epitop des Pre-M/M-Proteins eines Denguevirus bindet, wobei das Epitop wie in Anspruch 1 oder 2 definiert ist.

7. Pharmazeutische Zusammensetzung gegen Denguefieber, die einen Dengue-spezifischen Antikörper gemäß Anspruch 6 umfasst.

8. Pharmazeutische Zusammensetzung, die ein Denguevirus-spezifisches Peptid gemäß Anspruch 1 oder 2, das gegebenenfalls mit einer Trägersubstanz konjugiert ist, oder ein Fusionsprotein gemäß einem der Ansprüche 3 bis 5 umfasst.

9. Pharmazeutische Zusammensetzung gemäß Anspruch 8, die ein Adjuvans enthält.

10. Pharmazeutische Zusammensetzung gemäß Anspruch 8 oder 9, bei der es sich um einen Impfstoff gegen Denguefieber handelt.

## Revendications

1. Peptide spécifique du virus de la dengue comprenant un épitope de la protéine Pré-MM d'au moins un sérotype du virus de la dengue dans lequel ledit peptide est constitué des acides aminés n° 3 à 31, les acides aminés n° 69 à 93 ou les acides aminés n° 103 à 124 de Pré-MM.

2. Peptide spécifique du virus de la dengue selon la revendication 1, dans lequel ledit peptide comprend une séquence d'acides aminés choisie dans le groupe constitué de SEQ ID NO :1, SEQ ID : 4 et SEQ ID NO :5.

3. Protéine de fusion comprenant une protéine transporteur et une séquence d'acides aminés spécifique de la dengue comprenant un épitope du virus de la dengue-2 et/ou du virus de la dengue-4, dans lequel ladite séquence d'acides aminés spécifique de la dengue est constituée des acides aminés n° 1 à 42 ou les acides aminés n° 92 à 134 de la protéine Pré-MM dudit virus.

4. Protéine de fusion selon la revendication 3, dans laquelle la protéine transporteur est P64k de *Neisseria meningitidis* et la séquence d'acides aminés spécifique de la dengue est insérée après l'acide aminé n° 42 de P64k.

5. Protéine de fusion selon la revendication 4, comprenant une séquence choisie dans le groupe constitué de SEQ ID NO : 6, SEQ ID NO :7, SEQ ID NO :8 et SEQ IDE NO : 9.

6. Anticorps spécifique de la dengue se liant à un épitope de la protéine Pré-MM du virus de la dengue, ledit épitope étant tel que défini dans la revendication 1 ou 2.

7. Composition pharmaceutique contre la dengue, comprenant un anticorps spécifique du virus de la dengue tel que décrit dans la revendication 6.

8. Composition pharmaceutique comprenant un peptide spécifique du virus de la dengue selon la revendication 1 ou 2, éventuellement conjugué à une substance transporteur ou comprenant une protéine de fusion selon l'une quelconque des revendications 3 à 5.

9. Composition pharmaceutique selon la revendication 8, contenant un adjuvant.

10. Composition pharmaceutique selon la revendication 8 ou 9, qui est un vaccin contre la dengue.


### SEQUENCE LISTING

(1) GENERAL INFORMATION:
   (i) APPLICANT:
      (A) NAME: CENTRO DE INGENIERIA GENETICA Y BIOTECNOLOGIA
      (B) STREET: Ave. 31 entre 158 y 190, Playa
      (C) CITY: Ciudad de La Habana
      (E) COUNTRY: Cuba
      (F) POSTAL CODE (ZIP): 10600
      (G) TELEPHONE: 53 7 218466
      (H) TELEFAX: 53 7 218070 / 336008

      (A) NAME: INSTITUTO DE MEDICINA TROPICAL "PEDRO KOURI"
      (B) STREET: Autopista Novia del Mediodia Km 6, La Lisa
      (C) CITY: Ciudad de La Habana
      (E) COUNTRY: Cuba
      (F) POSTAL CODE (ZIP): 11100
      (G) TELEPHONE: 53 7 220633
      (H) TELEFAX: 53 7 335061
   (ii) TITLE OF INVENTION: PRE-M/M PROTEIN EPITOPES OF THE DENGUE VIRUS, SYNTHETIC PEPTIDES AND ITS USE.
   (iii) NUMBER OF SEQUENCES: 9
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
   (vi) PRIOR APPLICATION DATA:
      (A) APPLICATION NUMBER: CU 13/97
      (B) FILING DATE: 15-JAN-1997
(2) INFORMATION FOR SEQ ID NO: 1:
   (i) SEQUENCE CHARACTERISTICS:
      (A) LENGTH: 29 amino acids
      (B) TYPE: amino acid
      (C) STRANDEDNESS:
      (D) TOPOLOGY: linear
   (ii) MOLECULE TYPE: peptide
   (iii) HYPOTHETICAL: YES
   (iv) ANTI-SENSE: NO
   (v) FRAGMENT TYPE: internal
   (vi) ORIGINAL SOURCE:
      (A) ORGANISM: Dengue virus
      (B) STRAIN: Dengue-2
      (C) INDIVIDUAL ISOLATE: A-15
   (xi) SEQUENCE DESCRIPTION: SEQ ID NO: 1:
   Leu Thr Thr Arg Asn Gly Glu Pro His Met Ile Val Met Arg Gln Glu
   1 5 10 15
   Lys Gly Lys Ser Leu Leu Phe Lys Thr Gly Asp Gly Val
   20 25
(2) INFORMATION FOR SEQ ID NO: 2:
   (i) SEQUENCE CHARACTERISTICS:
      (A) LENGTH: 23 amino acids
      (B) TYPE: amino acid
      (C) STRANDEDNESS:
      (D) TOPOLOGY: linear
   (ii) MOLECULE TYPE: peptide
   (iii) HYPOTHETICAL: YES
   (iv) ANTI-SENSE: NO
   (v) FRAGMENT TYPE: internal
   (vi) ORIGINAL SOURCE:
      (A) ORGANISM: Dengue virus
      (B) STRAIN: Dengue-2
      (C) INDIVIDUAL ISOLATE: A-15
   (xi) SEQUENCE DESCRIPTION: SEQ ID NO: 2:
   Cys Glu Asp Thr Ile Thr Tyr Lys Cys Pro Leu Leu Arg Gln Asn Glu
   1 5 10 15
   Pro Glu Asp Ile Asp Cys Trp
   20
(2) INFORMATION FOR SEQ ID NO: 3:
   (i) SEQUENCE CHARACTERISTICS:
      (A) LENGTH: 36 amino acids
      (B) TYPE: amino acid
      (C) STRANDEDNESS:
      (D) TOPOLOGY: linear
   (ii) MOLECULE TYPE: peptide
   (iii) HYPOTHETICAL: YES
   (iv) ANTI-SENSE: NO
   (v) FRAGMENT TYPE: internal
   (vi) ORIGINAL SOURCE:
      (A) ORGANISM: Dengue virus
      (B) STRAIN: Dengue-2
      (C) INDIVIDUAL ISOLATE: A-15
   (xi) SEQUENCE DESCRIPTION: SEQ ID NO: 3:
   Arg Gln Asn Glu Pro Glu Asp Ile Asp Cys Trp Cys Asn Ser Thr Ser
   1               5                   10                  15
   Thr Trp Val Thr Tyr Gly Thr Cys Thr Thr Thr Gly Glu His Arg Arg
               20                  25                  30
   Glu Lys Arg Ser
           35
(2) INFORMATION FOR SEQ ID NO: 4:
   (i) SEQUENCE CHARACTERISTICS:
      (A) LENGTH: 25 amino acids
      (B) TYPE: amino acid
      (C) STRANDEDNESS:
      (D) TOPOLOGY: linear
   (ii) MOLECULE TYPE: peptide
   (iii) HYPOTHETICAL: YES
   (iv) ANTI-SENSE: NO
   (v) FRAGMENT TYPE: internal
   (vi) ORIGINAL SOURCE:
      (A) ORGANISM: Dengue virus
      (B) STRAIN: Dengue-2
      (C) INDIVIDUAL ISOLATE: A-15
   (xi) SEQUENCE DESCRIPTION: SEQ ID NO: 4:
   Asn Ser Thr Ser Thr Trp Val Thr Tyr Gly Thr Cys Thr Thr Thr Gly
   1               5                   10                  15
   Glu His Arg Arg Glu Lys Arg Ser Val
               20                  25
(2) INFORMATION FOR SEQ ID NO: 5:
   (i) SEQUENCE CHARACTERISTICS:
      (A) LENGTH: 22 amino acids
      (B) TYPE: amino acid
      (C) STRANDEDNESS:
      (D) TOPOLOGY: linear
   (ii) MOLECULE TYPE: peptide
   (iii) HYPOTHETICAL: YES
   (iv) ANTI-SENSE: NO
   (v) FRAGMENT TYPE: internal
   (vi) ORIGINAL SOURCE:
      (A) ORGANISM: Dengue virus
      (B) STRAIN: Dengue-2
      (C) INDIVIDUAL ISOLATE: A-15
   (xi) SEQUENCE DESCRIPTION: SEQ ID NO: 5:
   Leu Glu Thr Arg Thr Glu Thr Trp Met Ser Ser Glu Gly Ala Trp Lys
   1               5                   10                  15
   His Ala Gln Arg Ile Glu
               20
(2) INFORMATION FOR SEQ ID NO: 6:
   (i) SEQUENCE CHARACTERISTICS:
      (A) LENGTH: 635 amino acids
      (B) TYPE: amino acid
      (C) STRANDEDNESS:
      (D) TOPOLOGY: linear
   (ii) MOLECULE TYPE: protein
   (iii) HYPOTHETICAL: YES
   (iv) ANTI-SENSE: NO
   (vi) ORIGINAL SOURCE:
      (A) ORGANISM: Fusion protein
   (vii) IMMEDIATE SOURCE:
      (B) CLONE: PD31
   (xi) SEQUENCE DESCRIPTION: SEQ ID NO: 6:
   Met Ala Leu Val Glu Leu Lys Val Pro Asp Ile Gly Gly His Glu Asn
   1               5                   10                  15
   Val Asp Ile Ile Ala Val Glu Val Asn Val Gly Asp Thr Ile Ala Val
               20                  25                  30
   Asp Asp Thr Leu Ile Thr Leu Asp Leu Asp Phe His Leu Thr Thr Arg
           35                  40                  45
   Asn Gly Glu Pro His Met Ile Val Ser Arg Gln Glu Lys Gly Lys Ser
       50                  55                  60
   Leu Leu Phe Lys Thr Gly Asp Gly Val Asn Met Cys Thr Leu Met Ala
   65                  70                  75                  80
   Met Asp Leu Gly Leu Glu Met Asp Val Pro Ala Glu Val Ala Gly Val
                   85                  90                  95
   Val Lys Glu Val Lys Val Lys Val Gly Asp Lys Ile Ser Glu Gly Gly
               100                 105                 110
   Leu Ile Val Val Val Glu Ala Glu Gly Thr Ala Ala Ala Pro Lys Ala
           115                 120                 125
   Glu Ala Ala Ala Ala Pro Ala Gln Glu Ala Pro Lys Ala Ala Ala Pro
       130                 135                 140
   Ala Pro Gln Ala Ala Gln Phe Gly Gly Ser Ala Asp Ala Glu Tyr Asp
   145                 150                 155                 160
   Val Val Val Leu Gly Gly Gly Pro Gly Gly Tyr Ser Ala Ala Phe Ala
                   165                 170                 175
   Ala Ala Asp Glu Gly Leu Lys Val Ala Ile Val Glu Arg Tyr Lys Thr
               180                 185                 190
   Leu Gly Gly Val Cys Leu Asn Val Gly Cys Ile Pro Ser Lys Ala Leu
           195                 200                 205
   Leu His Asn Ala Ala Val Ile Asp Glu Val Arg His Leu Ala Ala Asn
       210                 215                 220
   Gly Ile Lys Tyr Pro Glu Pro Glu Leu Asp Ile Asp Met Leu Arg Ala
   225                 230                 235                 240
   Tyr Lys Asp Gly Val Val Ser Arg Leu Thr Gly Gly Leu Ala Gly Met
                   245                 250                 255
   Ala Lys Ser Arg Lys Val Asp Val Ile Gln Gly Asp Gly Gln Phe Leu
               260                 265                 270
   Asp Pro His His Leu Glu Val Ser Leu Thr Ala Gly Asp Ala Tyr Glu
           275                 280                 285
   Gln Ala Ala Pro Thr Gly Glu Lys Lys Ile Val Ala Phe Lys Asn Cys
       290                 295                 300
   Ile Ile Ala Ala Gly Ser Arg Val Thr Lys Leu Pro Phe Ile Pro Glu
   305                 310                 315                 320
   Asp Pro Arg Ile Ile Asp Ser Ser Gly Ala Leu Ala Leu Lys Glu Val
                   325                 330                 335
   Pro Gly Lys Leu Leu Ile Ile Gly Gly Gly Ile Ile Gly Leu Glu Met
               340                 345                 350
   Gly Thr Val Tyr Ser Thr Leu Gly Ser Arg Leu Asp Val Val Glu Met
           355                 360                 365
   Met Asp Gly Leu Met Gln Gly Ala Asp Arg Asp Leu Val Lys Val Trp
       370                 375                 380
   Gln Lys Gln Asn Glu Tyr Arg Phe Asp Asn Ile Met Val Asn Thr Lys
   385                 390                 395                 400
   Thr Val Ala Val Glu Pro Lys Glu Asp Gly Val Tyr Val Thr Phe Glu
                   405                 410                 415
   Gly Ala Asn Ala Pro Lys Glu Pro Gln Arg Tyr Asp Ala Val Leu Val
               420                 425                 430
   Ala Ala Gly Arg Ala Pro Asn Gly Lys Leu Ile Ser Ala Glu Lys Ala
           435                 440                 445
   Gly Val Ala Val Thr Asp Arg Gly Phe Ile Glu Val Asp Lys Gln Met
       450                 455                 460
   Arg Thr Asn Val Pro His Ile Tyr Ala Ile Gly Asp Ile Val Gly Gln
   465                 470                 475                 480
   Pro Met Leu Ala His Lys Ala Val His Glu Gly His Val Ala Ala Glu
                   485                 490                 495
   Asn Cys Ala Gly His Lys Ala Tyr Phe Asp Ala Arg Val Ile Pro Gly
               500                 505                 510
   Val Ala Tyr Thr Ser Pro Glu Val Ala Trp Val Gly Glu Thr Glu Leu
           515                 520                 525
   Ser Ala Lys Ala Ser Gly Arg Lys Ile Thr Lys Ala Asn Phe Pro Trp
       530                 535                 540
   Ala Ala Ser Gly Arg Ala Ile Ala Asn Gly Cys Asp Lys Pro Phe Thr
   545                 550                 555                 560
   Lys Leu Ile Phe Asp Ala Glu Thr Gly Arg Ile Ile Gly Gly Gly Ile
                   565                 570                 575
   Val Gly Pro Asn Gly Gly Asp Met Ile Gly Glu Val Cys Leu Ala Ile
               580                 585                 590
   Glu Met Gly Cys Asp Ala Ala Asp Ile Gly Lys Thr Ile His Pro His
           595                 600                 605
   Pro Thr Leu Gly Glu Ser Ile Gly Met Ala Ala Glu Val Ala Leu Gly
       610                 615                 620
   Thr Cys Thr Asp Leu Pro Pro Gln Lys Lys Lys
   625                 630                 635
(2) INFORMATION FOR SEQ ID NO: 7:
   (i) SEQUENCE CHARACTERISTICS:
      (A) LENGTH: 635 amino acids
      (B) TYPE: amino acid
      (C) STRANDEDNESS:
      (D) TOPOLOGY: linear
   (ii) MOLECULE TYPE: protein
   (iii) HYPOTHETICAL: YES
   (iv) ANTI-SENSE: NO
   (vi) ORIGINAL SOURCE:
      (A) ORGANISM: Fusion protein
   (vii) IMMEDIATE SOURCE:
      (B) CLONE: PD30
   (xi) SEQUENCE DESCRIPTION: SEQ ID NO: 7:
   Met Ala Leu Val Glu Leu Lys Val Pro Asp Ile Gly Gly His Glu Asn
   1               5                   10                  15
   Val Asp Ile Ile Ala Val Glu Val Asn Val Gly Asp Thr Ile Ala Val
               20                  25                  30
   Asp Asp Thr Leu Ile Thr Leu Asp Leu Glu Ser Val Ala Leu Val Pro
           35                  40                  45
   His Val Gly Met Gly Leu Glu Thr Arg Thr Glu Thr Trp Met Ser Ser
       50                  55                  60
   Glu Gly Ala Trp Lys His Ala Gln Arg Ile Glu Thr Trp Ile Leu Arg
   65                  70                  75                  80
   His Pro Gly Phe Leu Glu Met Asp Val Pro Ala Glu Val Ala Gly Val
                   85                  90                  95
   Val Lys Glu Val Lys Val Lys Val Gly Asp Lys Ile Ser Glu Gly Gly
               100                 105                 110
   Leu Ile Val Val Val Glu Ala Glu Gly Thr Ala Ala Ala Pro Lys Ala
           115                 120                 125
   Glu Ala Ala Ala Ala Pro Ala Gln Glu Ala Pro Lys Ala Ala Ala Pro
       130                 135                 140
   Ala Pro Gln Ala Ala Gln Phe Gly Gly Ser Ala Asp Ala Glu Tyr Asp
   145                 150                 155                 160
   Val Val Val Leu Gly Gly Gly Pro Gly Gly Tyr Ser Ala Ala Phe Ala
                   165                 170                 175
   Ala Ala Asp Glu Gly Leu Lys Val Ala Ile Val Glu Arg Tyr Lys Thr
               180                 185                 190
   Leu Gly Gly Val Cys Leu Asn Val Gly Cys Ile Pro Ser Lys Ala Leu
           195                 200                 205
   Leu His Asn Ala Ala Val Ile Asp Glu Val Arg His Leu Ala Ala Asn
       210                 215                 220
   Gly Ile Lys Tyr Pro Glu Pro Glu Leu Asp Ile Asp Met Leu Arg Ala
   225                 230                 235                 240
   Tyr Lys Asp Gly Val Val Ser Arg Leu Thr Gly Gly Leu Ala Gly Met
                   245                 250                 255
   Ala Lys Ser Arg Lys Val Asp Val Ile Gln Gly Asp Gly Gln Phe Leu
               260                 265                 270
   Asp Pro His His Leu Glu Val Ser Leu Thr Ala Gly Asp Ala Tyr Glu
           275                 280                 285
   Gln Ala Ala Pro Thr Gly Glu Lys Lys Ile Val Ala Phe Lys Asn Cys
       290                 295                 300
   Ile Ile Ala Ala Gly Ser Arg Val Thr Lys Leu Pro Phe Ile Pro Glu
   305                 310                 315                 320
   Asp Pro Arg Ile Ile Asp Ser Ser Gly Ala Leu Ala Leu Lys Glu Val
                   325                 330                 335
   Pro Gly Lys Leu Leu Ile Ile Gly Gly Gly Ile Ile Gly Leu Glu Met
               340                 345                 350
   Gly Thr Val Tyr Ser Thr Leu Gly Ser Arg Leu Asp Val Val Glu Met
           355                 360                 365
   Met Asp Gly Leu Met Gln Gly Ala Asp Arg Asp Leu Val Lys Val Trp
       370                 375                 380
   Gln Lys Gln Asn Glu Tyr Arg Phe Asp Asn Ile Met Val Asn Thr Lys
   385                 390                 395                 400
   Thr Val Ala Val Glu Pro Lys Glu Asp Gly Val Tyr Val Thr Phe Glu
                   405                 410                 415
   Gly Ala Asn Ala Pro Lys Glu Pro Gln Arg Tyr Asp Ala Val Leu Val
               420                 425                 430
   Ala Ala Gly Arg Ala Pro Asn Gly Lys Leu Ile Ser Ala Glu Lys Ala
           435                 440                 445
   Gly Val Ala Val Thr Asp Arg Gly Phe Ile Glu Val Asp Lys Gln Met
       450                 455                 460
   Arg Thr Asn Val Pro His Ile Tyr Ala Ile Gly Asp Ile Val Gly Gln
   465                 470                 475                 480
   Pro Met Leu Ala His Lys Ala Val His Glu Gly His Val Ala Ala Glu
                   485                 490                 495
   Asn Cys Ala Gly His Lys Ala Tyr Phe Asp Ala Arg Val Ile Pro Gly
               500                 505                 510
   Val Ala Tyr Thr Ser Pro Glu Val Ala Trp Val Gly Glu Thr Glu Leu
           515                 520                 525
   Ser Ala Lys Ala Ser Gly Arg Lys Ile Thr Lys Ala Asn Phe Pro Trp
       530                 535                 540
   Ala Ala Ser Gly Arg Ala Ile Ala Asn Gly Cys Asp Lys Pro Phe Thr
   545                 550                 555                 560
   Lys Leu Ile Phe Asp Ala Glu Thr Gly Arg Ile Ile Gly Gly Gly Ile
                   565                 570                 575
   Val Gly Pro Asn Gly Gly Asp Met Ile Gly Glu Val Cys Leu Ala Ile
               580                 585                 590
   Glu Met Gly Cys Asp Ala Ala Asp Ile Gly Lys Thr Ile His Pro His
           595                 600                 605
   Pro Thr Leu Gly Glu Ser Ile Gly Met Ala Ala Glu Val Ala Leu Gly
       610                 615                 620
   Thr Cys Thr Asp Leu Pro Pro Gln Lys Lys Lys
   625                 630                 635
(2) INFORMATION FOR SEQ ID NO: 8:
   (i) SEQUENCE CHARACTERISTICS:
      (A) LENGTH: 677 amino acids
      (B) TYPE: amino acid
      (C) STRANDEDNESS:
      (D) TOPOLOGY: linear
   (ii) MOLECULE TYPE: protein
   (iii) HYPOTHETICAL: YES
   (iv) ANTI-SENSE: NO
   (vi) ORIGINAL SOURCE:
      (A) ORGANISM: Fusion protein
   (vii) IMMEDIATE SOURCE:
      (B) CLONE: PD33
   (xi) SEQUENCE DESCRIPTION: SEQ ID NO: 8:
   Met Ala Leu Val Glu Leu Lys Val Pro Asp Ile Gly Gly His Glu Asn
   1               5                   10                  15
   Val Asp Ile Ile Ala Val Glu Val Asn Val Gly Asp Thr Ile Ala Val
               20                  25                  30
   Asp Asp Thr Leu Ile Thr Leu Asp Leu Glu Ser Val Ala Leu Val Pro
   35 40 45
   His Val Gly Met Gly Leu Glu Thr Arg Thr Glu Thr Trp Met Ser Ser
   50 55 60
   Glu Gly Ala Trp Lys His Ala Gln Arg Ile Glu Thr Trp Ile Leu Arg
   65 70 75 80
   His Pro Gly Phe Ser Val Ala Leu Thr Pro His Ser Gly Met Gly Leu
   85 90 95
   Glu Thr Arg Ala Glu Thr Trp Met Ser Ser Glu Gly Ala Trp Lys His
   100 105 110
   Ala Gln Arg Val Glu Ser Trp Ile Leu Arg Asn Pro Arg Phe Leu Glu
   115 120 125
   Met Asp Val Pro Ala Glu Val Ala Gly Val Val Lys Glu Val Lys Val
   130 135 140
   Lys Val Gly Asp Lys Ile Ser Glu Gly Gly Leu Ile Val Val Val Glu
   145 150 155 160
   Ala Glu Gly Thr Ala Ala Ala Pro Lys Ala Glu Ala Ala Ala Ala Pro
   165 170 175
   Ala Gln Glu Ala Pro Lys Ala Ala Ala Pro Ala Pro Gln Ala Ala Gln
   180 185 190
   Phe Gly Gly Ser Ala Asp Ala Glu Tyr Asp Val Val Val Leu Gly Gly
   195 200 205
   Gly Pro Gly Gly Tyr Ser Ala Ala Phe Ala Ala Ala Asp Glu Gly Leu
   210 215 220
   Lys Val Ala Ile Val Glu Arg Tyr Lys Thr Leu Gly Gly Val Cys Leu
   225 230 235 240
   Asn Val Gly Cys Ile Pro Ser Lys Ala Leu Leu His Asn Ala Ala Val
   245 250 255
   Ile Asp Glu Val Arg His Leu Ala Ala Asn Gly Ile Lys Tyr Pro Glu
   260 265 270
   Pro Glu Leu Asp Ile Asp Met Leu Arg Ala Tyr Lys Asp Gly Val Val
   275 280 285
   Ser Arg Leu Thr Gly Gly Leu Ala Gly Met Ala Lys Ser Arg Lys Val
   290 295 300
   Asp Val Ile Gln Gly Asp Gly Gln Phe Leu Asp Pro His His Leu Glu
   305 310 315 320
   Val Ser Leu Thr Ala Gly Asp Ala Tyr Glu Gln Ala Ala Pro Thr Gly
   325 330 335
   Glu Lys Lys Ile Val Ala Phe Lys Asn Cys Ile Ile Ala Ala Gly Ser
   340 345 350
   Arg Val Thr Lys Leu Pro Phe Ile Pro Glu Asp Pro Arg Ile Ile Asp
   355 360 365
   Ser Ser Gly Ala Leu Ala Leu Lys Glu Val Pro Gly Lys Leu Leu Ile
   370 375 380
   Ile Gly Gly Gly Ile Ile Gly Leu Glu Met Gly Thr Val Tyr Ser Thr
   385                 390                 395                 400
   Leu Gly Ser Arg Leu Asp Val Val Glu Met Met Asp Gly Leu Met Gln
                   405                 410                 415
   Gly Ala Asp Arg Asp Leu Val Lys Val Trp Gln Lys Gln Asn Glu Tyr
               420                 425                 430
   Arg Phe Asp Asn Ile Met Val Asn Thr Lys Thr Val Ala Val Glu Pro
           435                 440                 445
   Lys Glu Asp Gly Val Tyr Val Thr Phe Glu Gly Ala Asn Ala Pro Lys
       450                 455                 460
   Glu Pro Gln Arg Tyr Asp Ala Val Leu Val Ala Ala Gly Arg Ala Pro
   465                 470                 475                 480
   Asn Gly Lys Leu Ile Ser Ala Glu Lys Ala Gly Val Ala Val Thr Asp
                   485                 490                 495
   Arg Gly Phe Ile Glu Val Asp Lys Gln Met Arg Thr Asn Val Pro His
               500                 505                 510
   Ile Tyr Ala Ile Gly Asp Ile Val Gly Gln Pro Met Leu Ala His Lys
           515                 520                 525
   Ala Val His Glu Gly His Val Ala Ala Glu Asn Cys Ala Gly His Lys
       530                 535                 540
   Ala Tyr Phe Asp Ala Arg Val Ile Pro Gly Val Ala Tyr Thr Ser Pro
   545                 550                 555                 560
   Glu Val Ala Trp Val Gly Glu Thr Glu Leu Ser Ala Lys Ala Ser Gly
                   565                 570                 575
   Arg Lys Ile Thr Lys Ala Asn Phe Pro Trp Ala Ala Ser Gly Arg Ala
               580                 585                 590
   Ile Ala Asn Gly Cys Asp Lys Pro Phe Thr Lys Leu Ile Phe Asp Ala
           595                 600                 605
   Glu Thr Gly Arg Ile Ile Gly Gly Gly Ile Val Gly Pro Asn Gly Gly
       610                 615                 620
   Asp Met Ile Gly Glu Val Cys Leu Ala Ile Glu Met Gly Cys Asp Ala
   625                 630                 635                 640
   Ala Asp Ile Gly Lys Thr Ile His Pro His Pro Thr Leu Gly Glu Ser
                   645                 650                 655
   Ile Gly Met Ala Ala Glu Val Ala Leu Gly Thr Cys Thr Asp Leu Pro
               660                 665                 670
   Pro Gln Lys Lys Lys
           675
(2) INFORMATION FOR SEQ ID NO: 9:
   (i) SEQUENCE CHARACTERISTICS:
      (A) LENGTH: 635 amino acids
      (B) TYPE: amino acid
      (C) STRANDEDNESS:
      (D) TOPOLOGY: linear
   (ii) MOLECULE TYPE: protein
   (iii) HYPOTHETICAL: YES
   (iv) ANTI-SENSE: NO
   (vi) ORIGINAL SOURCE:
      (A) ORGANISM: Fusion protein
   (vii) IMMEDIATE SOURCE:
      (B) CLONE: PD34
   (xi) SEQUENCE DESCRIPTION: SEQ ID NO: 9:
   Met Ala Leu Val Glu Leu Lys Val Pro Asp Ile Gly Gly His Glu Asn
   1               5                   10                  15
   Val Asp Ile Ile Ala Val Glu Val Asn Val Gly Asp Thr Ile Ala Val
               20                  25                  30
   Asp Asp Thr Leu Ile Thr Leu Asp Leu Glu Ser Val Ala Leu Thr Pro
           35                  40                  45
   His Ser Gly Met Gly Leu Glu Thr Arg Ala Glu Thr Trp Met Ser Ser
       50                  55                  60
   Glu Gly Ala Trp Lys His Ala Gln Arg Val Glu Ser Trp Ile Leu Arg
   65                  70                  75                  80
   Asn Pro Arg Phe Leu Glu Met Asp Val Pro Ala Glu Val Ala Gly Val
                   85                  90                  95
   Val Lys Glu Val Lys Val Lys Val Gly Asp Lys Ile Ser Glu Gly Gly
               100                 105                 110
   Leu Ile Val Val Val Glu Ala Glu Gly Thr Ala Ala Ala Pro Lys Ala
           115                 120                 125
   Glu Ala Ala Ala Ala Pro Ala Gln Glu Ala Pro Lys Ala Ala Ala Pro
       130                 135                 140
   Ala Pro Gln Ala Ala Gln Phe Gly Gly Ser Ala Asp Ala Glu Tyr Asp
   145                 150                 155                 160
   Val Val Val Leu Gly Gly Gly Pro Gly Gly Tyr Ser Ala Ala Phe Ala
                   165                 170                 175
   Ala Ala Asp Glu Gly Leu Lys Val Ala Ile Val Glu Arg Tyr Lys Thr
               180                 185                 190
   Leu Gly Gly Val Cys Leu Asn Val Gly Cys Ile Pro Ser Lys Ala Leu
           195                 200                 205
   Leu His Asn Ala Ala Val Ile Asp Glu Val Arg His Leu Ala Ala Asn
       210                 215                 220
   Gly Ile Lys Tyr Pro Glu Pro Glu Leu Asp Ile Asp Met Leu Arg Ala
   225                 230                 235                 240
   Tyr Lys Asp Gly Val Val Ser Arg Leu Thr Gly Gly Leu Ala Gly Met
                   245                 250                 255
   Ala Lys Ser Arg Lys Val Asp Val Ile Gln Gly Asp Gly Gln Phe Leu
               260                 265                 270
   Asp Pro His His Leu Glu Val Ser Leu Thr Ala Gly Asp Ala Tyr Glu
           275                 280                 285
   Gln Ala Ala Pro Thr Gly Glu Lys Lys Ile Val Ala Phe Lys Asn Cys
       290                 295                 300
   Ile Ile Ala Ala Gly Ser Arg Val Thr Lys Leu Pro Phe Ile Pro Glu
   305                 310                 315                 320
   Asp Pro Arg Ile Ile Asp Ser Ser Gly Ala Leu Ala Leu Lys Glu Val
                   325                 330                 335
   Pro Gly Lys Leu Leu Ile Ile Gly Gly Gly Ile Ile Gly Leu Glu Met
               340                 345                 350
   Gly Thr Val Tyr Ser Thr Leu Gly Ser Arg Leu Asp Val Val Glu Met
           355                 360                 365
   Met Asp Gly Leu Met Gln Gly Ala Asp Arg Asp Leu Val Lys Val Trp
       370                 375                 380
   Gln Lys Gln Asn Glu Tyr Arg Phe Asp Asn Ile Met Val Asn Thr Lys
   385                 390                 395                 400
   Thr Val Ala Val Glu Pro Lys Glu Asp Gly Val Tyr Val Thr Phe Glu
                   405                 410                 415
   Gly Ala Asn Ala Pro Lys Glu Pro Gln Arg Tyr Asp Ala Val Leu Val
               420                 425                 430
   Ala Ala Gly Arg Ala Pro Asn Gly Lys Leu Ile Ser Ala Glu Lys Ala
           435                 440                 445
   Gly Val Ala Val Thr Asp Arg Gly Phe Ile Glu Val Asp Lys Gln Met
       450                 455                 460
   Arg Thr Asn Val Pro His Ile Tyr Ala Ile Gly Asp Ile Val Gly Gln
   465                 470                 475                 480
   Pro Met Leu Ala His Lys Ala Val His Glu Gly His Val Ala Ala Glu
                   485                 490                 495
   Asn Cys Ala Gly His Lys Ala Tyr Phe Asp Ala Arg Val Ile Pro Gly
               500                 505                 510
   Val Ala Tyr Thr Ser Pro Glu Val Ala Trp Val Gly Glu Thr Glu Leu
           515                 520                 525
   Ser Ala Lys Ala Ser Gly Arg Lys Ile Thr Lys Ala Asn Phe Pro Trp
       530                 535                 540
   Ala Ala Ser Gly Arg Ala Ile Ala Asn Gly Cys Asp Lys Pro Phe Thr
   545                 550                 555                 560
   Lys Leu Ile Phe Asp Ala Glu Thr Gly Arg Ile Ile Gly Gly Gly Ile
                   565                 570                 575
   Val Gly Pro Asn Gly Gly Asp Met Ile Gly Glu Val Cys Leu Ala Ile
               580                 585                 590
   Glu Met Gly Cys Asp Ala Ala Asp Ile Gly Lys Thr Ile His Pro His
           595                 600                 605
   Pro Thr Leu Gly Glu Ser Ile Gly Met Ala Ala Glu Val Ala Leu Gly
       610                 615                 620
   Thr Cys Thr Asp Leu Pro Pro Gln Lys Lys Lys
   625                 630                 635